# EUROPEAN PATENT SPECIFICATION

(11) **EP 2 821 054 B1**
(45) Date of publication and mention of the grant of the patent: **31.01.2018**
(21) Application number: 13755570.2
(22) Date of filing: 01.03.2013
(51) Int. Cl.: A61K 8/36, A61K 8/39, A61K 8/41, A61K 8/45, A61K 8/86, A61Q 19/10, C11D 1/04, C11D 1/06

(54) **SKIN CLEANSING COMPOSITION**
HAUTREINIGUNGSZUSAMMENSETZUNG
COMPOSITION NETTOYANTE POUR LA PEAU

(30) Priority: 02.03.2012 JP 2012047079
(43) Date of publication of application: 07.01.2015
(73) Proprietor: Kao Corporation, Chuo-ku Tokyo 103-8210 (JP)
(72) Inventor: MASUI, Takashi, Tokyo 131-8501 (JP); TAKEUCHI, Hiroki, Tokyo 131-8501 (JP); YAMADA, Shinji, Haga-gun Tochigi 321-3497 (JP)
(74) Representative: Hoffmann Eitle
(86) International application number: PCT/JP2013/055686
(87) International publication number: WO 2013/129652

(56) References cited:
- EP-A1- 2 821 053
- WO-A1-2011/071079
- WO-A1-2012/029950
- WO-A1-2013/085035
- JP-A- H0 625 695
- JP-A- S6 197 395
- JP-A- H05 112 795
- JP-A- 2005 112 946
- JP-A- 2011 140 485
- JP-A- 2011 178 681
- US-A1- 2009 023 623
- NIKKO CHEMICALS CO., LTD. ET AL.: 'Keshohin Handbook', 01 November 1996 pages 163 - 165, XP008174613

## Description

### [Field of the Invention]

The present invention relates to a skin cleansing composition.

### [Background of the Invention]

Soaps have long been used as skin cleansers for reasons such as their excellent lathering performance. However, soaps create a strong sense of specific friction during rinsing, which occasionally caused consumers to think of a negative impact on the skin. That is, friction gave consumers the impression that "oil of the skin has been completely removed" or "there is some residual feel," etc.

Also, although alkyl ether carboxylic acid-based surfactants are known to be gentle to the skin, they have poor foaming properties; therefore, the use of these surfactants in combination with other surfactants such as alkyl ether sulfate is studied (Patent Publication 1). Further, cleansing compositions comprising ether carboxylic acid-based surfactants having a narrow molecular weight distribution (Patent Publications 2 and 3), a cleanser composition comprising an ether carboxylic acid-based surfactant having a specific distribution of moles of ethylene oxide added (Patent Publication 4), and the like have also been proposed.

### Citation List

### [Patent Publication]

[Patent Publication 1] JP-A-2008-285479
[Patent Publication 2] JP-A-S61-21199
[Patent Publication 3] JP-A-2001-207189
[Patent Publication 4] JP-A-H02-175799

### [Summary of the Invention]

The present invention relates to a skin cleansing composition, comprising the following components (A) and (B) :
(A) from 0.1 to 30% by mass of an alkyl ether carboxylic acid or a salt thereof represented by the formula (1):

   R¹-O-(CH₂CH₂O)ₙ-CH₂-COOM (1)

   wherein, R¹ represents an alkyl group having 8 to 18 carbon atoms, n represents a number of from 0 to 20, and
   M represents a hydrogen atom, alkali metal, alkaline earth metal, ammonium, or organic ammonium, wherein R¹ contains 2 or more alkyl groups, and the content of a component having an alklyl chain length which is contained in the highest content is 55% by mass or more and less than 97% by mass,
   wherein, R¹ has an average carbon number of from 10.8 to 12.8 and an average value of n is from 2.5 to 3.4,
   and wherein, the alkyl ether carboxylic acid or the salt thereof contains a component in which n = 0 in an amount of from 9.6 to 27% by mass, and a component in which n = 1 and a component in which n = 2 in a total amount of 27% by mass to 36.5% by mass, and
(B) from 0.1 to 30% by mass of a potassium salt or an alkanolamine salt of a fatty acid represented by the formula (2):

   R²-COOY (2)

   wherein, R² represents an alkyl group or an alkenyl group having 9 to 22 carbon atoms, and Y represents potassium or alkanolamine.

### [Brief Description of the Drawings]

[Figure 1] Figure 1 is a set of diagrams illustrating an apparatus and a method for measuring friction behavior during rinsing when the skin cleansing composition of the present invention was used in Examples.
[Figure 2] Figure 2 is a diagram illustrating friction behavior during rinsing when the skin cleansing composition of Example 1 was used.
[Figure 3] Figure 3 is a diagram illustrating friction behavior during rinsing when the skin cleansing composition of Comparative Example 2 was used.
[Figure 4] Figure 4 is a diagram illustrating friction behavior during rinsing when the skin cleansing composition of Comparative Example 8 was used.
[Figure 5] Figure 5 is a diagram illustrating friction behavior during rinsing when the skin cleansing composition of Comparative Example 6 was used.
[Figure 6] Figure 6 is a diagram illustrating friction behavior when there is slipperiness during rinsing.
[Figure 7] Figure 7 is a diagram illustrating friction behavior when there is squeakiness (overly-intense friction) during rinsing.
[Figure 8] Figure 8 is a diagram illustrating friction behavior when favorable rinse feel with reduced residual feel is obtained. Favorable rinse feel means that during rinsing after washing is performed with the cleansing composition of the present invention, hands move smoothly when they are sliding on the skin, but the hands feel themselves getting caught and stopped when they make a turn in a reciprocatory rubbing movement.
[Figure 9] Figure 9 is a diagram illustrating friction behavior during rinsing when the skin cleansing composition of Example 2 was used.
[Figure 10] Figure 10 is a diagram illustrating friction behavior during rinsing when the skin cleansing composition of Example 3 was used.
[Figure 11] Figure 11 is a diagram illustrating friction behavior during rinsing when the skin cleansing composition of Example 4 was used.
[Figure 12] Figure 12 is a
   diagram illustrating friction behavior during rinsing when the skin cleansing composition of Example 5 was used.
[Figure 13] Figure 13 is a diagram illustrating friction behavior during rinsing when the skin cleansing composition of Example 6 was used.
[Figure 14] Figure 14 is a diagram illustrating friction behavior during rinsing when the skin cleansing composition of Example 7 was used.
[Figure 15] Figure 15 is a diagram illustrating friction behavior during rinsing when the skin cleansing composition of Example 8 was used.

### [Detailed Description of the Invention]

The cleansing compositions of Patent Publications 1 to 4 also produce a small volume of foam and give a slippery feel during rinsing, which occasionally gave consumers the impression that cleansers were still left on the skin.

The present invention relates to a skin cleansing composition which produces a large volume of foam with excellent foam quality and gives favorable rinse feel with reduced residual feel.

The present inventors have found that a skin cleansing composition which produces a large volume of foam with excellent foam quality and gives favorable rinse feel with reduced residual feel can be obtained by using an alkyl ether carboxylic acid having a specific distribution or a salt thereof in combination with a specific fatty acid salt.

The skin cleansing composition of the present invention produces a large volume of foam with excellent foam quality, and moreover, gives favorable rinse feel with reduced residual feel. More specifically, the skin cleansing composition of the present invention gives no slippery feel during rinsing, during which the skin is repeatedly rubbed with hands to rinse off foam and cleansers in running water, and while the skin is rubbed with hands, hands are less likely to feel themselves getting caught and squeaky (overly-intense friction). That is, the skin cleansing composition of the present invention achieves a characteristic rinse feel, meaning that hands move smoothly when they are sliding on the skin, but the hands feel themselves getting caught and stopped when they make a turn in a reciprocatory rubbing movement.

The alkyl ether carboxylic acid or the salt thereof of the component (A) used in the present invention is represented by the formula (1).

In the formula, R¹ is an alkyl group having 8 to 18 carbon atoms, preferably an alkyl group having 8 to 16 carbon atoms, and further preferably an alkyl group having 10 to 16 carbon atoms. Also, although the alkyl chain of R¹ may be either linear or branched, from the viewpoint of foaming properties, a linear alkyl group is preferred. Also, R¹ has an average carbon number of from 10.8 to 12.8, preferably from 10.8 to 12.5, and more preferably from 12.1 to 12.4. It is preferable that the average carbon number be within the above range since excellent foaming properties, foam qualities, and stability at low temperature are obtained.

Also, R¹ contains two or more alkyl groups, and the content of a component having the alkyl chain length contained therein with the highest content is 55% by mass or more and less than 97% by mass, preferably from 60 to 95% by mass, and even more preferably from 70 to 95% by mass since excellent volume of foam and foam qualities are obtained.

Also, in the formula, n represents a number of from 0 to 20, and preferably from 0 to 12. It is to be noted that n represents the number of moles of ethylene oxide added, and the average number of moles of ethylene oxide added in the composition of the component (A) (an average value of n) is from 2.5 to 3.4, preferably from 2.8 to 3.4, and further preferably from 2.8 to 3.1 since favorable foam qualities are achieved.

The alkyl ether carboxylic acid or the salt thereof of the component (A) contains, in the formula (1), a component in which n = 0 in an amount of from 9.6 to 27% by mass, and preferably from 9.9 to 16% by mass, and further preferably from 9.9 to 15% by mass. When the content of the component in which n = 0 is within the above range, the feeling of friction during rinsing is improved.

Further, the total content of a component in which n = 1 and a component in which n = 2 is 27% by mass to 36.5% by mass, preferably from 35 to 36.1% by mass.

Also, in the formula, examples of M include a hydrogen atom; alkali metal such as sodium and potassium; alkaline earth metal such as calcium and magnesium; ammonium; alkanolamine-derived ammonium such as monoethanolamine, diethanolamine, and triethanolamine. Among them, alkali metal is preferred, and potassium is more preferred in terms of foaming properties, stability at low temperature, and absence of coloration over time.

In the formula (1), the alkyl ether carboxylic acid or the salt thereof of the component (A) preferably has a mass ratio of the components in which n = 0, 1, 2, 3, and 4, (the mass of a component in which n = 0) : (the mass of a component in which n = 1) : (the mass of a component in which n = 2) : (the mass of a component in which n = 3) : (the mass of a component in which n = 4), of 1 : 0.99 to 3.50 : 0.89 to 3.00 : 0.76 to 3.00 : 0.63 to 1.6 in a component having the alkyl chain length contained with the highest content in a composition of R¹ from the viewpoint that foaming properties, detergency, and a feeling of being stopped during rinsing can be achieved simultaneously.

Also, in the formula (1), it is preferable that the content of a component in which n = 0 be 9.6% by mass or more and less than 12% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52 in a component having the alkyl chain length contained with the highest content in a composition of R¹, or it is preferable that the content of a component in which n = 0 be from 12 to 17% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99 in a component having the alkyl chain length contained with the highest content in a composition of R¹ from the viewpoint of foaming properties and rinsing properties.

Further, in the formula (1), it is preferable that the content of a component in which n = 0 be from 9.9 to 11.5% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 1.58 to 1.84 : 1.72 to 2.17 : 1.49 to 2.00 : 1.14 to 1.52 in a component having the alkyl chain length contained with the highest content in a composition of R¹, or it is preferable that the content of a component in which n = 0 be from 13 to 17% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 1.00 to 1.31 : 0.93 to 1.34 : 0.79 to 1.18 : 0.63 to 0.99 in a component having the alkyl chain length contained with the highest content in a composition of R¹ from the viewpoint of volume of foam, foam qualities, and rinsing properties.

In the component (A), in the formula (1), R¹ is an alkyl group having 8 to 18 carbon atoms, R¹ has an average carbon number of 10.8 to 12.8, and the content of a component having the alkyl chain length contained therein with the highest content be 55% by mass or more and less than 97% by mass, and further, n represents a number of from 0 to 20 and the average value thereof be from 2.5 to 3.4, and a component in which n = 0 is contained in an amount of from 9.6 to 27% by mass, and a component in which n = 1 and a component in which n = 2 be contained in a total amount of from 27 to 36.5% by mass. Also, as M in the formula, a hydrogen atom, sodium, potassium, and ammonium are preferred, and potassium is more preferred. A cleansing composition comprising the alkyl ether carboxylic acid or the salt thereof having the aforementioned configuration allows hands to move smoothly while they are sliding on the skin, and a sense of being stopped which is felt as the hands make a turn in a reciprocatory rubbing movement to be intensified, during rinsing.

It is preferable that, in the component (A), in the formula (1), R¹ be an alkyl group having 8 to 16 carbon atoms, R¹ has an average carbon number of 10.8 to 12.5, and a content of a component having the alkyl chain length contained therein with the highest content be from 60 to 95% by mass, and further, n represents a number of from 0 to 20 and an average value thereof be from 2.8 to 3.4, and a component in which n = 0 be contained in an amount of from 9.6 to 27% by mass, preferably from 9.9 to 16% by mass, and a component in which n = 1 and a component in which n = 2 be contained in a total amount of from 27 to 36.5% by mass. Also, as M in the formula, a hydrogen atom, sodium, potassium, and ammonium are preferred, and potassium is more preferred. A cleansing composition comprising the alkyl ether carboxylic acid or the salt thereof having the aforementioned configuration can have improved volume of foam and foam qualities.

It is preferable that, in the component (A), in the formula (1), R¹ be an alkyl group having 10 to 16 carbon atoms, R¹ has an average carbon number of from 12.1 to 12.4, and the content of a component having the alkyl chain length which is contained in the highest content be from 60 to 95% by mass, and further, n represents a number of from 0 to 20 and the average value thereof be from 2.8 to 3.1, and a component in which n = 0 be contained in an amount of from 9.9 to 15% by mass, and a component in which n = 1 and a component in which n = 2 be contained in a total amount of from 35 to 36.1% by mass. Also, as M in the formula, a hydrogen atom, sodium, potassium, and ammonium are preferred, and potassium is more preferred. A cleansing composition comprising the alkyl ether carboxylic acid or the salt thereof having the aforementioned configuration can have improved volume of foam, foam qualities and favorable rinse feel with reduced residual feel.

It is to be noted that in the component (A) of the present invention, the distribution of the alkyl chain length of R¹, the average alkyl chain length of R¹, the amount of a component in which n = 0, the average number of added moles n, and a mass ratio of the components in which n = 0, 1, 2, 3, and 4 are obtained as follows from the gas chromatographic analysis of the alkyl ether carboxylic acid represented by the formula (1).

### [Distribution of the alkyl chain length of R¹]

From the peak areas obtained by gas chromatography, a peak area of each alkyl chain length corresponding to n = 0 mole was obtained, and setting the sum of the peak areas thus obtained at 100, the percentage of the distribution of each alkyl chain length was calculated. Similar calculation was carried out also as to n = 1 to 3 moles, and the percentage values of the distribution of each alkyl chain length corresponding to n = 0 to 3 moles were averaged out, whereby the distribution of the alkyl chain length of R¹ was obtained (from this, the alkyl group component contained in the largest amount in the composition of R¹ can be specified).

### [Average alkyl chain length of R¹]

From the distribution of the alkyl chain length of R¹ obtained as above, the proportion of each component was obtained, which was multiplied by the number of carbon atoms of the corresponding alkyl chain length, and the resulting values were summed up. The value thus obtained was used as an average alkyl chain length.

### [Amount of a component in which n = 0, total content of a component in which n = 1 and a component in which n = 2]

In the composition of R¹, the alkyl chain length contained therein with the highest content was specified, and the peak areas of the component having alkyl chain length of the highest content corresponding to n = 0 to 10 were added up by gas chromatography. By setting the total amount thus obtained at 100%, the amount of a component in which n = 0, the total content of a component in which n = 1 and a component in which n = 2 were calculated.

### [Average number of added moles n]

In the composition of R¹, the alkyl chain length of the highest content was specified, and the peak areas of the component having the alkyl chain length of the highest content corresponding to n = 0 to 10 were added up by gas chromatography (the amount of a component in which n is 11 or more was so small that it was excluded from the calculation). By setting the total amount thus obtained at 1, each proportion of n = 0 to 10 was obtained. The resulting proportion was multiplied by each number of added moles, and the sum of the resulting values was used as the average number of added moles n.

### [Mass ratio of the components in which n = 0, 1, 2, 3, and 4]

As to the ratio of each of the components having different numbers of moles of EO added, the distribution of the alkyl chain length of R¹ was obtained from the peak area obtained by gas chromatography by the method described above, and the component having the alkyl chain length of the highest content in the composition of R¹ was specified, and the ratio of each of the components having different numbers of moles of EO added was specified by the area ratio of n = 0, n = 1, n = 2, n = 3, and n = 4 of the component having the alkyl chain length of the highest content.

The alkyl ether carboxylic acid or the salt thereof of the component (A) has the aforementioned composition, and from the viewpoint of rinse feel, the content thereof is 0.1% by mass or more, preferably 0.5% by mass or more, more preferably 1% by mass or more and 30% by mass or less, preferably 0.5% by mass or less, and more preferably 15% by mass or less of the total composition. Further, the content of the component (A) is from 0.1 to 30% by mass, preferably from 0.5 to 20% by mass, and more preferably from 1 to 15% by mass of the total composition.

The fatty acid salt of the component (B) used in the present invention is represented by the aforementioned formula (2).

In the formula, R² represents an alkyl group or an alkenyl group having 9 to 22 carbon atoms, which may be linear or branched. R² is preferably an alkyl group having 11 to 17 carbon atoms.

Also, Y represents potassium or alkanolamine. Examples of the alkanolamine include monoethanolamine, diethanolamine, and triethanolamine. Among them, potassium is more preferable.

The component (B) can be used alone or in combination of two or more thereof, and from the viewpoint of foaming properties and the volume of foam, the content thereof is 0.1% by mass or more, preferably 0.5% by mass or more, more preferably 1% by mass or more and 30% by mass or less, preferably 20% by mass or less, and more preferably 15% by mass or less of the total composition. Further, the content of the component (B) is from 0.1 to 30% by mass, preferably from 0.5 to 20% by mass, and more preferably from 1 to 15% by mass of the total composition.

From the viewpoint of easy handling, the total content of the components (A) and (B) in the skin cleansing composition of the present invention is preferably 0.5% by mass or more, more preferably 1% by mass or more, even more preferably 5% by mass or more and preferably 40% by mass or less, more preferably 30% by mass or less, and even more preferably 15% by mass or less of the total composition. Also, the total content of the components (A) and (B) is preferably from 0.5 to 40% by mass, more preferably from 1 to 30% by mass, and even more preferably from 5 to 15% by mass of the total composition.

Also, the mass ratio of the component (A) to the component (B) in the cleansing composition of the present invention is preferably (A) : (B) = 1 : 10 to 10 : 1, more preferably (A) : (B) = 1 : 5 to 5 : 1, and even more preferably (A) : (B) = 1 : 2 to 2 : 1.

It should be noted that the total degree of neutralization of the components (A) and (B) is preferably larger than 0.9 equivalent, and preferably 1.2 equivalent or less, more preferably 1 equivalent or less.

When only the component (A) is added to the cleansing composition, rinsing becomes difficult due to slipperiness in the step of rinsing after washing the body. Meanwhile, when only the component (B) is added to the cleansing composition, an overly-intense sense of friction is created and some residual feel is imparted in the step of rinsing.

According to the present invention, by using the component (A) in combination with the component (B), a large volume of foam with excellent foam quality is produced and favorable rinse feel with reduced residual feel is obtained. Favorable rinse feel means that during rinsing, hands move smoothly when they are sliding on the skin, but the hands feel themselves getting caught and stopped when they make a turn in a reciprocatory rubbing movement.

The cleansing composition of the present invention can further comprise (C) an amphoteric surfactant, thereby further improving the volume of foam and foam quality without affecting rinse feel.

Examples of the component (C) include an acetic acid betaine surfactant such as lauryl dimethylaminoacetic acid betaine, an amine oxide surfactant such as lauryldimethylamine oxide, an imidazolinium betaine surfactant such as 2-alkyl-N-carboxymethyl-N-hydroxyethyl imidazolinium betaine, an amidebetaine surfactant such as lauric acid amidopropyl betaine, and a sulfobetaine surfactant such as lauryl hydroxy sulfobetaine.

Among them, from the viewpoint of foaming properties, lauric acid amidopropyl betaine and lauryl hydroxy sulfobetaine are preferable.

The component (C) can be used alone or in combination of two or more thereof, and from the viewpoint of foaming properties, the content thereof is preferably 0.1% by mass or more, more preferably 0.5% by mass or more, even more preferably 1% by mass or more and preferably 30% by mass or less, more preferably 20% by mass or less, and even more preferably 15% by mass or less of the total composition. Further, the content of the component (C) is preferably from 0.1 to 30% by mass, more preferably from 0.5 to 20% by mass, and even more preferably from 1 to 15% by mass of the total composition.

Also, from the viewpoint of foaming properties, the mass ratio of the component (A) to the component (C) is preferably (A) : (C) = 2 : 1 to 1 : 2, and more preferably 2 : 1 to 1 : 1.

The skin cleansing composition of the present invention can further comprise water as a solvent. The content of water is preferably from 10 to 94.5% by mass, and more preferably from 15 to 90% by mass of the total composition. Water is added as balance of the cleansing composition other than the aforementioned components and other components composing the cleansing composition.

As a preferable embodiment of the present invention, there is provided a skin cleansing composition, comprising the following components (A) and (B):
(A) an alkyl ether carboxylic acid or a salt thereof represented by the formula (1):

   R¹-O-(CH₂CH₂O)ₙ-CH₂-COOM (1)

   wherein, R¹ represents an alkyl group having 8 to 18 carbon atoms, n represents a number of from 0 to 20, and M represents a hydrogen atom, alkali metal, alkaline earth metal, ammonium, or organic ammonium,
   wherein, R¹ has an average carbon number of from 10.8 to 12.8, and an average value of n is from 2.5 to 3.4, preferably from 2.8 to 3.4,
   and wherein, the alkyl ether carboxylic acid or the salt thereof contains a component in which n = 0 in an amount of from 12 to 17% by mass, preferably from 13 to 17% by mass and a component in which n = 1 and a component in which n = 2 in a total amount of from 27 to 36.5% by mass and has a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99, preferably 1 : 1.00 to 1.31 : 0.93 to 1.34 : 0.79 to 1.18 : 0.63 to 0.99 in a component having the alkyl chain length contained with the highest content in a composition of R¹, and (B) a potassium salt or an alkanolamine salt of a fatty acid represented by the formula (2):

   R²-COOY (2)

   wherein, R² represents an alkyl group or an alkenyl group having 9 to 22 carbon atoms, and Y represents potassium or alkanolamine,
   wherein, the mass ratio of the component (A) to the component (B) is (A) : (B) = 1 : 5 to 5 : 1.

The skin cleansing composition of the present invention may further comprise components used in ordinary cleansers such as surfactants other than the aforementioned components, humectants, oil components, disinfecting agents, anti-inflammatory agents, antiseptic agents, chelating agents, thickening agents, salts, pearlescent agents, scrub agents, fragrances, cooling agents, dyes, ultraviolet absorbers, antioxidants, and plant extracts.

The skin cleansing composition of the present invention is produced by mixing the blending components by a routine method. The cleansing composition thus obtained may be either a liquid or a solid, and preferably a liquid aqueous formulation. When it is a liquid, the viscosity at 25°C as measured by a B-type viscometer (manufactured by Tokyo Keiki Inc.) is preferably from 200 to 80000 mPa·s. The viscosity can be adjusted by appropriately selecting the blending components.

Also, the pH is preferably from 3 to 12, and more preferably from 5 to 10.5. Also, the value of pH is measured in each cleanser composition diluted 20-fold with ion exchange water at 25°C.

The skin cleansing composition of the present invention is suitable as, for example, a face wash, a body soap, and a hand soap.

The skin cleansing method using the skin cleansing composition of the present invention is exemplified as follows. That is, there is provided a method comprising applying an adequate amount of the skin cleansing composition of the present invention to the body, namely the body's skin areas such as face, hands, feet, and torso, lathering up and washing, and then rinsing off using warm water from a shower and the like. It is also possible to use a washing aid such as a towel, a sponge, and a brush to allow the cleansing composition of the present invention to lather up for washing.

In connection with the aforementioned embodiments, the present invention further discloses the following compositions, production methods and applications.
<1> A skin cleansing composition, comprising the following components (A) and (B):
   (A) from 0.1 to 30% by mass of an alkyl ether carboxylic acid or a salt thereof represented by the formula (1):

      R¹-O- (CH₂CH₂O)ₙ-CH₂-COOM (1)

      wherein, R¹ represents an alkyl group having 8 to 18 carbon atoms, n represents a number of from 0 to 20, and M represents a hydrogen atom, alkali metal, alkaline earth metal, ammonium, or organic ammonium,
      wherein, R¹ has an average carbon number of from 10.8 to 12.8 and an average value of n is from 2.5 to 3.4,
      and wherein, the alkyl ether carboxylic acid or the salt thereof contains a component in which n = 0 in an amount of from 9.6 to 27% by mass, and a component in which n = 1 and a component in which n = 2 in a total amount of 27% by mass or more to 36.5% by mass, and
   (B) from 0.1 to 30% by mass of a potassium salt or an alkanolamine salt of a fatty acid represented by the formula (2):

      R²-COOY (2)

      wherein, R² represents an alkyl group or an alkenyl group having 9 to 22 carbon atoms, and Y represents potassium or alkanolamine.
<2> The skin cleansing composition according to the aforementioned <1>, wherein, in the component (A), in the formula (1), R¹ is preferably an alkyl group having 8 to 16 carbon atoms, and more preferably an alkyl group having 10 to 16 carbon atoms.
<3> The skin cleansing composition according to the aforementioned <1> or <2>, wherein, in the component (A), in the formula (1), an average carbon number of R¹ is preferably from 10.8 to 12.5, and more preferably from 12.1 to 12.4.
<4> The skin cleansing composition according to any one of the aforementioned <1> to <3>, wherein, in the component (A), in the formula (1), an average value of n is preferably from 2.8 to 3.4, and more preferably from 2.8 to 3.1.
<5> The skin cleansing composition according to any one of the aforementioned <1> to <4>, wherein, in the component (A), in the formula (1), a component in which n = 0 is contained in an amount of preferably from 9.9 to 16% by mass, and more preferably from 9.9 to 15% by mass.
<6> The skin cleansing composition according to any one of the aforementioned <1> to <5>, wherein, in the component (A), in the formula (1), the total content of a component in which n = 1 and a component in which n = 2 is preferably from 35 to 36.1% by mass.
<7> The skin cleansing composition according to any one of the aforementioned <1> to <6>, wherein, in the component (A), in the formula (1), the content of a component in which n = 0 is preferably from 9.9 to 27% by mass.
<8> The skin cleansing composition according to any one of the aforementioned <1> to <7>, wherein, in the component (A), in the formula (1), R¹ is preferably an alkyl group having 8 to 16 carbon atoms, an average value of n is preferably from 2.8 to 3.4, and the content of a component in which n = 0 is preferably from 9.9 to 27% by mass.
<9> The skin cleansing composition according to any one of the aforementioned <1> to <8>, wherein, the component (A) has, preferably, in the formula (1), a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 3.50 : 0.89 to 3.00 : 0.76 to 3.00 : 0.63 to 1.52 in a component having the alkyl chain length contained with the highest content in a composition of R¹.
<10> The skin cleansing composition according to any one of the aforementioned <1> to <9>, wherein, in the component (A), in the formula (1), R¹ preferably contains two or more alkyl groups, and the content of a component having an alkyl chain length which is contained in the highest content is preferably from 60 to 95% by mass, and more preferably from 70 to 95% by mass.
<11> The skin cleansing composition according to any one of the aforementioned <1> to <10>, wherein, the component (A) contains, in the formula (1), a component in which n = 0 in an amount of 9.6% by mass or more and less than 12% by mass and has a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52 in a component having the alkyl chain length contained with the highest content in a composition of R¹, or contains a component in which n = 0 in an amount of from 12 to 17% by mass and has a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99 in a component having the alkyl chain length contained with the highest content in a composition of R¹.
<12> The skin cleansing composition according to any one of the aforementioned <1> to <11>, wherein the content of the component (A) is preferably from 0.5 to 20% by mass, and more preferably from 1 to 15% by mass.
<13> The skin cleansing composition according to any one of the aforementioned <1> to <12>, wherein, in the component (B), in the formula (2), R² is preferably an alkyl group having 11 to 17 carbon atoms, and Y is preferably potassium.
<14> The skin cleansing composition according to any one of the aforementioned <1> to <13>, wherein the content of the component (B) is preferably from 0.5 to 20% by mass, and more preferably from 1 to 15% by mass.
<15> The skin cleansing composition according to any one of the aforementioned <1> to <14>, wherein a mass ratio of the component (A) to the component (B) is preferably (A) : (B) = 1 : 10 to 10 : 1, more preferably 1 : 5 to 5 : 1, and even more preferably 1 : 2 to 2 : 1.
<16> The skin cleansing composition according to any one of the aforementioned <1> to <15>, wherein the total content of the component (A) and the component (B) is preferably from 0.5 to 40% by mass, more preferably from 1 to 30% by mass, and even more preferably from 5 to 15% by mass of the total composition.
<17> The skin cleansing composition according to any one of the aforementioned <1> to <16>, further comprising (C) an amphoteric surfactant.
<18> The skin cleansing composition according to the aforementioned <17>, wherein the amphoteric surfactant of the component (C) is preferably an acetic acid betaine surfactant, an amine oxide surfactant, an imidazolinium betaine surfactant, an amidebetaine surfactant, or a sulfobetaine surfactant.
<19> The skin cleansing composition according to the aforementioned <17> or <18>, wherein the amphoteric surfactant of the component (C) is preferably lauric acid amidopropyl betaine or lauryl hydroxy sulfobetaine.
<20> The skin cleansing composition according to any one of the aforementioned <17> to <19>, wherein the content of the component (C) is preferably from 0.1 to 30% by mass, more preferably from 0.5 to 20% by mass, and even more preferably from 1 to 15% by mass of the total composition.
<21> The skin cleansing composition according to any one of the aforementioned <17> to <20>, wherein a mass ratio of the component (A) to the component (C) is preferably (A) : (C) = 2 : 1 to 1 : 2, and more preferably 2 : 1 to 1 : 1.
<22> The skin cleansing composition according to any one of the aforementioned <1> to <21>, wherein the content of water is preferably from 10 to 94.5% by mass, and more preferably from 15 to 90% by mass of the total composition.
<23> The skin cleansing composition according to any one of the aforementioned <1> to <22>, wherein a total degree of neutralization of the components (A) and (B) is preferably larger than 0.9 equivalent, and preferably 1.2 equivalent or less, more preferably 1 equivalent or less.
<24> A skin cleansing method, comprising applying the skin cleansing composition according to any one of the aforementioned <1> to <23> to a skin area, washing, and then rinsing.
<25> Use of the composition according to any one of the aforementioned <1> to <23> as a skin cleanser.

### <Measurement method>

In the present invention, the alkyl composition, the distribution of the moles of EO added, and the ratio of each component of the alkyl ether carboxylic acid were measured by the following analytical method with gas chromatography (GC).

Unless otherwise noted, "%" represents % by mass.

### (GC analytical conditions)

GC instrument; the product of Agilent Technologies, 7890A Column; the product of Agilent Technologies, DB-5 (30 m, an inner diameter of 0.25 mm, a film thickness of 0.25 µm)
Detector; FID
Carrier; helium gas, 1 mL/min
Conditions of temperature rising; temperature is raised at 5°C/min from 100°C to 325°C, and thereafter, maintained at 325°C for 35 minutes.

### (Method of sample pretreatment)

Into 50 mL of methanol, 150 mg of alkyl ether carboxylic acid was dissolved. Also, the cleansing composition was taken in an amount of 150 mg in terms of alkyl ether carboxylic acid equivalent and dissolved in 50 mL of methanol. Also, when the cleansing composition contained a strong anionic surfactant such as polyoxyethylene alkyl ether sulfate, the cleansing composition was collected in such an amount that the strong anionic surfactant was 250 mg or less. From these solutions, 1 mL was taken and applied to a solid phase cartridge (manufactured by Biotage Japan Ltd., Isolute SAX, 1 g, 3 mL, 500-0100-B) which had been conditioned with 4 mL of methanol in advance, and the filtrate was received in a 10 mL round-bottom test tube. Subsequently, the eluate which was eluted with 6 mL of a solution of 4.6 g of formic acid in 100 mL of methanol was also collected in the same test tube. The solution thus collected was set in a block heater heated to 50°C, to which nitrogen gas was blown in, and the solution was concentrated to approximately 1 mL, which was then dried at room temperature by further blowing nitrogen gas. To the resulting product, 2 mL of a diazomethane-ether solution was added, and the resulting solution was left to stand at room temperature for 10 minutes while stirring to carry out derivatization. Subsequently, nitrogen gas was blown in at room temperature and the solution was concentrated to 500 µL or less, to which chloroform was then added to bring the total volume to 500 µL, and the resulting product was subjected to GC analysis.

It is to be noted that the diazomethane-ether solution was prepared by the following procedure using a diazomethane generator (manufactured by Miyamoto Riken Ind. Co., Ltd., GM-50). A first receiver and a second receiver, and the second receiver and a third receiver were connected using a silicone rubber plug and a Teflon (Registered trademark) tube. Into the second receiver, 0.8 g of N-methyl-N'-nitro-N-nitrosoguanidine was collected, to which 2.5 mL of ion exchange water was added. Into the third receiver, 10 mL of tert-butyl methyl ether was collected. The first, second, and third receivers were cooled on ice. Subsequently, the second receiver was equipped with a plastic syringe, into which 3 mL of a solution of 20 g of sodium hydroxide dissolved in 100 mL of ion exchange water was placed. This aqueous solution of sodium hydroxide was slowly added dropwise to generate diazomethane gas, and nitrogen gas was gently blown in from the first receiver side to dissolve the diazomethane gas in tert-butyl methyl ether in the third receiver, whereby a diazomethane-ether solution was obtained.

The following reagents were used in the aforementioned sample pretreatment.
Methanol (manufactured by Kanto Chemical Co., Inc., for high performance liquid chromatography, 25183-1B) Formic acid (manufactured by Wako Pure Chemical Industries, Ltd., special grade chemical, 066-00461) Chloroform (manufactured by Kanto Chemical Co., Inc., CICA first grade, 07278-01)
N-Methyl-N'-nitro-N-nitrosoguanidine (manufactured by Kanto Chemical Co., Inc., CICA first grade, 25596-51) Tert-butyl methyl ether (manufactured by Kanto Chemical Co., Inc., CICA special grade, 04418-00)
Sodium hydroxide (manufactured by Wako Pure Chemical Industries, Ltd., special grade, 196-13761)

### <Production Example>

The alkyl ether carboxylic acid of the component (A) used in the skin cleansing composition of the present invention is obtainable by a production method of alkyl ether carboxylic acid, which includes reacting ethylene oxide with one or two or more alcohols selected from alcohols having an alkyl group having 8 to 18 carbon atoms to obtain alkyl ethoxylate, and then subjecting the alkyl ethoxylate thus obtained to further reactions. Specifically, the alkyl ether carboxylic acid of the component (A) can be produced, for example, as follows. Also, unless otherwise noted, "%" represents % by mass.

### Production Example 1

Into a stainless steel autoclave with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.68 g (0.0478 mol) of potassium hydroxide were placed and dehydration was performed under reduced pressure. Subsequently, 996 g (22.6 mol) of ethylene oxide (EO) was introduced at 155°C and reactions were allowed to proceed at a reaction temperature of 155°C and a reaction pressure of 0.4 MPa for two hours. Upon completion of the reaction, the resulting mixture was stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 3.55 moles of EO added (hereinbelow, also referred to as "the produced AE") was obtained.

Into a glass reaction container with stirring and temperature controlling functions and an oxygen gas introduction tube, 90 g (0.2 mol) of the aforementioned product, 16.7 g of a 48% aqueous solution of sodium hydroxide (0.2 mol as sodium hydroxide), 0.9 g of a palladium-platinum-bismuth-based catalyst (activated carbon containing 4% of palladium, 1% of platinum, 5% of bismuth, and 50% of water content), and 494.4 g of water were each placed. While stirring, the liquid temperature was raised to 70°C, and while blowing oxygen in at a ratio of 27 mol% (with respect to the produced AE / hour), catalytic oxidation reactions were carried out at a reaction temperature of 70°C for 3.5 hours. The rate of reaction was 89%.

Upon completion of the reaction, the catalyst was filtered out from the reaction solution to provide an aqueous solution of sodium salt of alkyl ether carboxylic acid. Subsequently, 35% hydrochloric acid was added, and a liquid separation operation was performed to provide alkyl ether carboxylic acid, which will be referred to as EC1.

As a result of gas chromatography analysis, it was found that, in the formula (1), M = H, R¹ had lauryl group / myristyl group at a ratio of 95 / 5, the average carbon number was 12.1, and the average value of n was 2.8, and EC1 contained a component in which n = 0 in an amount of 14.7% by mass, and a component in which n = 1 and a component in which n = 2 in a total amount of 36.1% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.22 : 1.23 : 1.06 : 0.83.

### Production Example 2

According to Production Example 1, EO was reacted with a raw material containing a mixture of decyl alcohol [trade name: KALCOL 1098, manufactured by Kao Corporation], lauryl alcohol [trade name: KALCOL 2098, manufactured by Kao Corporation], myristyl alcohol [trade name: KALCOL 4098, manufactured by Kao Corporation], and cetyl alcohol [trade name: KALCOL 6098, manufactured by Kao Corporation] at a mass ratio of 10 / 70 / 15 / 5 to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ had decyl group/lauryl group/myristyl group/ palmityl group at a ratio of 10 / 70 / 15 / 5, the average carbon number was 12.3, and the average value of n was 3.3, and the alkyl ether carboxylic acid contained a component in which n = 0 in an amount of 15.2% by mass, and a component in which n = 1 and a component in which n = 2 in a total amount of 31.4% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.07 : 1.00 : 0.85 : 0.67.

### Production Example 3

Into a glass reaction container with stirring and temperature controlling functions, 372 g (2.00 mol) of lauryl alcohol was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, the precipitates were filtered out. Subsequently, 35% hydrochloric acid was added for acidification to obtain alkyl ether carboxylic acid (in the formula (1), M = H, R¹ was a lauryl group, and n = 0).

### Production Example 4

According to Production Example 1, EO was reacted with decyl alcohol as a raw material to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a decyl group, and the average value of n was 3.1, and the alkyl ether carboxylic acid contained a component in which n = 0 in an amount of 16% by mass, and a component in which n = 1 and a component in which n = 2 in a total amount of 37% by mass.

### Production Example 5

According to Production Example 1, EO was reacted with lauryl alcohol as a raw material to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a lauryl group, and the average value of n was 3.1, and the alkyl ether carboxylic acid contained a component in which n = 0 in an amount of 16% by mass, and a component in which n = 1 and a component in which n = 2 in a total amount of 37% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.19 : 1.13 : 0.94 : 1.

### Production Example 6 (Reference)

According to Production Example 1, EO was reacted with myristyl alcohol as a raw material to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a myristyl group, and the average value of n was 3.1, and the alkyl ether carboxylic acid contained a component in which n = 0 in an amount of 16% by mass, and a component in which n = 1 and a component in which n = 2 in a total amount of 37% by mass.

### Production Example 7 (Reference)

According to Production Example 1, EO was added to a raw material containing a mixture of lauryl alcohol and cetyl alcohol at a mass ratio of 20 / 80 to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ had lauryl group / palmityl group at a ratio of 20 / 80, and the average value of n was 3.1, and the alkyl ether carboxylic acid contained a component in which n = 0 in an amount of 16% by mass, and a component in which n = 1 and a component in which n = 2 in a total amount of 37% by mass.

### Production Example 8 (Reference)

According to Production Example 1, EO was reacted with lauryl alcohol as a raw material to provide alkyl ethoxylate having 4.05 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a lauryl group, the average value of n was 3.5, and the resulting alkyl ether carboxylic acid contained a component in which n = 0 in an amount of 11.4% by mass, and a component in which n = 1 and a component in which n = 2 in a total amount of 30.6% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.31 : 1.38 : 1.25 : 1.06.

### Production Example 9

Into a stainless steel autoclave with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.6 g (0.0478 mol) of potassium hydroxide were placed and dehydration was performed under reduced pressure. Subsequently, 718 g (16.3 mol) of ethylene oxide (EO) was introduced at 155°C and a reaction was allowed to proceed at a reaction temperature of 155°C and a reaction pressure of 0.4 MPa for two hours. Upon completion of the reaction, the resulting mixture was cooled and then stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 2.55 moles of EO added was obtained.

Into a glass reaction container with stirring and temperature controlling functions, 600 g (2.00 mol) of the aforementioned product was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, 35% hydrochloric acid was added for acidification until pH was 2.8, and the resulting oil layer was collected to obtain alkyl ether carboxylic acid, which will be referred to as EC6.

As a result of gas chromatography analysis, it was found that, in the formula (1), M = H, R¹ had lauryl group / myristyl group at a ratio of 94 / 6, the average carbon number was 12.1, and the average value of n was 3.1, and EC6 contained a component in which n = 0 in an amount of 9.9% by mass, and a component in which n = 1 and a component in which n = 2 in a total amount of 35.4% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.65 : 1.92 : 1.74 : 1.32.

In the Examples, EC2 was obtained by mixing each of the alkyl ether carboxylic acids produced in Production Examples 5, 6, and 7 at a mass ratio of 78.75 / 15 / 6.25.

In the Examples, EC3 was obtained by mixing each of the alkyl ether carboxylic acid obtained in Production Examples 2 and 3 at a mass ratio of 90 / 10.

In the Examples, EC4 was obtained by mixing each of EC1 obtained in Production Example 1 and the alkyl ether carboxylic acid obtained in Production Example 4 at a mass ratio of 40 / 60.

In the Examples, EC5 was obtained by mixing each of the alkyl ether carboxylic acid obtained in Production Examples 2 and 8 at a mass ratio of 40 / 60.

### [Examples]

### Examples 1 to 39 and Comparative Examples 1 to 13

The skin cleansing compositions having the compositions as shown in Tables 3 to 6 were produced, and the volume of foam, foam qualities, and rinse feel were evaluated. The results are shown in Tables 3 to 6 altogether.

Also, the composition of the component (A) used in Examples is as shown in Tables 1 and 2.

Also, for the average numbers of moles of EO added in commercially available alkyl ether carboxylic acids used in Examples and Comparative Examples (AKYPO RLM25 (manufactured by Kao Corporation), AKYPO RLM45 (manufactured by Kao Corporation), AKYPO RLM100NV (manufactured by Kao Corporation), BEAULIGHT LCA (manufactured by Sanyo Chemical Industries, Ltd.), and ECTD-3NEX (manufactured by Nikko Chemicals, Co., Ltd)), values provided in the catalogue supplied by each vendor or values posted in the website of each vendor were referred to. Unknown alkyl composition, the amount of a component in which n = 0, and the total amount of a component in which n = 1 and a component in which n = 2 were analyzed by the aforementioned method.

### (Production method)

To a portion of water, the component (A) and a 48% aqueous solution of potassium hydroxide, a 48% aqueous solution of sodium hydroxide, arginine, triethanolamine, and aminomethyl propanol were added in an amount equal to 1.0 equivalent of the component (A). The resulting mixture was heated to 70°C and homogenized. To a portion of water, potassium laurate or potassium myristate of the component (B) was added, and then the resulting mixture was heated to 80°C and homogenized. The resulting solutions of the components (A) and (B) and further, when necessary, the component (C) were mixed in accordance with the compositions shown in Tables 3 to 6. Water was further added to adjust the solutions to predetermined concentrations, followed by cooling to 15°C to 35°C, whereby skin cleansing compositions were obtained.

### (Evaluation method)

### (1) Volume of foam:

A lathering mesh (manufactured by Daisan Co. of Hakugen Group) was moistened with water (approximately 8 g). Then, 1 g of each skin cleansing composition was placed on the lathering mesh. The lathering mesh was held in both hands in an enveloping manner and the hands were moved in a circular motion for lathering. The hands were rubbed together in a circular motion 40 times for lathering. The foam produced with the lathering mesh was collected in a 500 mL beaker (manufactured by AGC Techno Glass Co., Ltd., 8.5 cm in diameter and 15 cm in height). After condensing the foam by shaking the beaker, the height (cm) of the foam thus collected is measured with a ruler. Then, the volume (cm³) of the foam thus collected was calculated from the height thus obtained and the bottom surface area of the beaker.

### (2) Foam qualities:

One expert panelist organoleptically evaluated the foam produced in (1) based on the following criteria.
A; Foam is very soft and fine.
B; Foam is soft and fine.
C; Foam is hard, making washing difficult.
D; Foam has bubbly foam qualities, making washing difficult.

### (3) Rinse feel:

The foam produced in (1) was spread over the entire forearm and rubbed 10 times in a back and forth motion to wash the arm, followed by rinsing with tap water (approximately 30°C). At this time, the rinse feel was organoleptically evaluated by one expert panelist based on the following criteria.
A; While hands move smoothly when they are sliding on the skin, they feel themselves getting caught when they make a turn in a reciprocatory rubbing movement, and non-slippery, very excellent rinse feel is obtained without residual feel.
B; While hands move smoothly when they are sliding on the skin, they feel themselves getting caught when they make a turn in a reciprocatory rubbing movement, and non-slippery, favorable rinse feel is obtained without residual feel.
C; Poor rinse feel with a strong friction sense and residual feel.
D; Rinsing was difficult with slipperiness and residual feel.

**[Table 1]**

| | R¹ (% by mass) | | | | Average carbon number | Average number of moles of EO added | n=0 Content ratio | n=1, 2 Total amount |
|---|---|---|---|---|---|---|---|---|
| | C10 | C12 | C14 | C16 | | | | |
| EC1 | 0 | 95 | 5 | 0 | 12.1 | 2.8 | 14.7% | 36.1% |
| EC2 | 0 | 80 | 15 | 5 | 12.5 | 3.1 | 16.0% | 32.5% |
| EC3 | 8 | 73 | 13.5 | 4.5 | 12.2 | 2.82 | 27.0% | 27.1% |
| EC4 | 60 | 38 | 2 | 0 | 10.8 | 3.2 | 12.5% | 34.8% |
| EC5 | 4 | 88 | 6 | 2 | 12.1 | 3.4 | 13.3% | 31.0% |
| EC6 | 0 | 94 | 6 | 0 | 12.1 | 3.1 | 9.9% | 35.4% |

**[Table 2]**

| | R¹ (% by mass) | | | | Average carbon number | Average number of moles of EO added | n=4 Content ratio | n=1, 2 Total amount |
|---|---|---|---|---|---|---|---|---|
| | C10 | C12 | C14 | C16 | | | | |
| 25CA *1 | 0 | 68 | 26 | 6 | 12.8 | 2.5 | 16.0% | 32.5% |
| 100NV *2 | 0 | 68 | 26 | 6 | 12.8 | 10 | 4.9% | 20.4% |
| LCA *3 | 0 | 100 | 0 | 0 | 12 | 3 | 2.8% | 42.8% |
| Nex *4 | - | - | - | - | C13 branched | 3 | 4.2% | 40.0% |

| | | | | | | | | |
|---|---|---|---|---|---|---|---|---|
| *1: AKYPO RLM25 (manufactured by Kao Corporation) *2: AKYPO RLM100NV (manufactured by Kao Corporation) *3: BEAULIGHT LCA (manufactured by Sanyo Chemical Industries, Ltd.) *4: ECTD-3NEX (manufactured by Nikko Chemicals, Co., Ltd) | | | | | | | | |

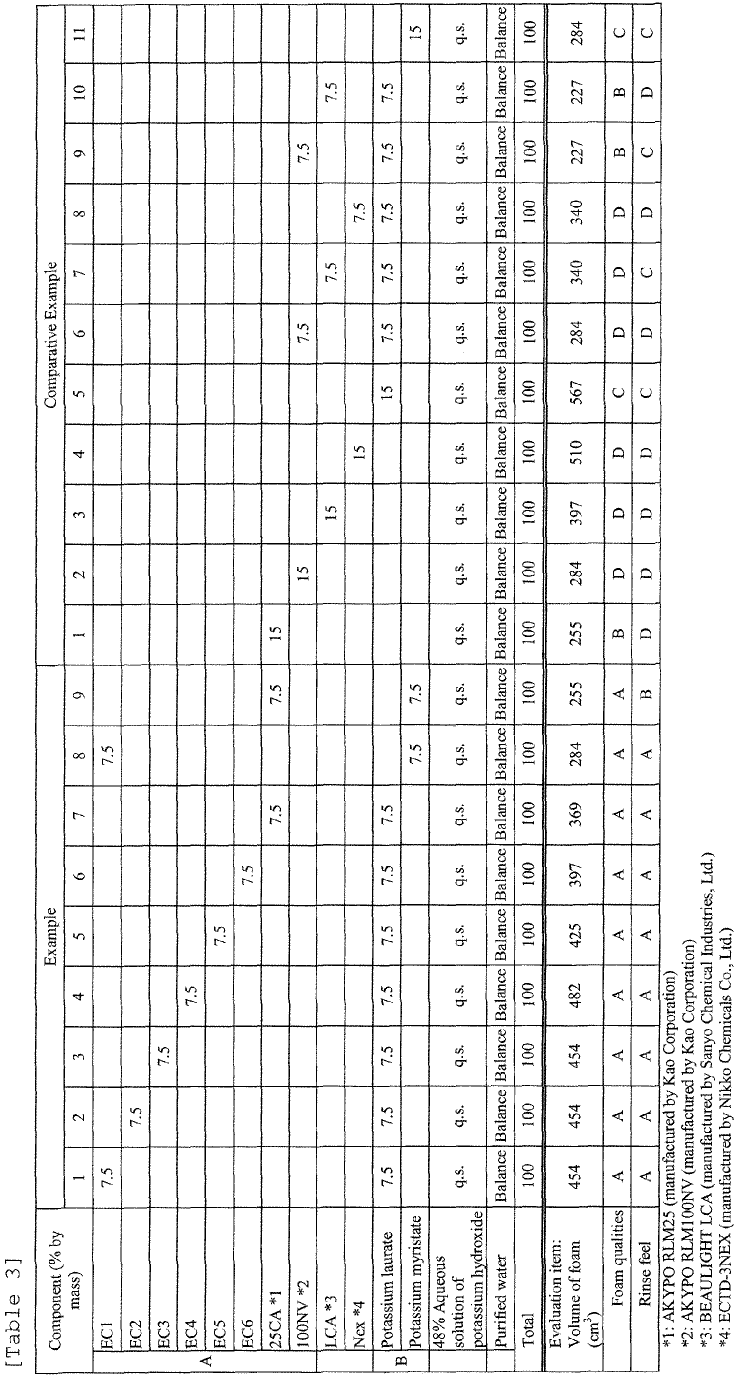

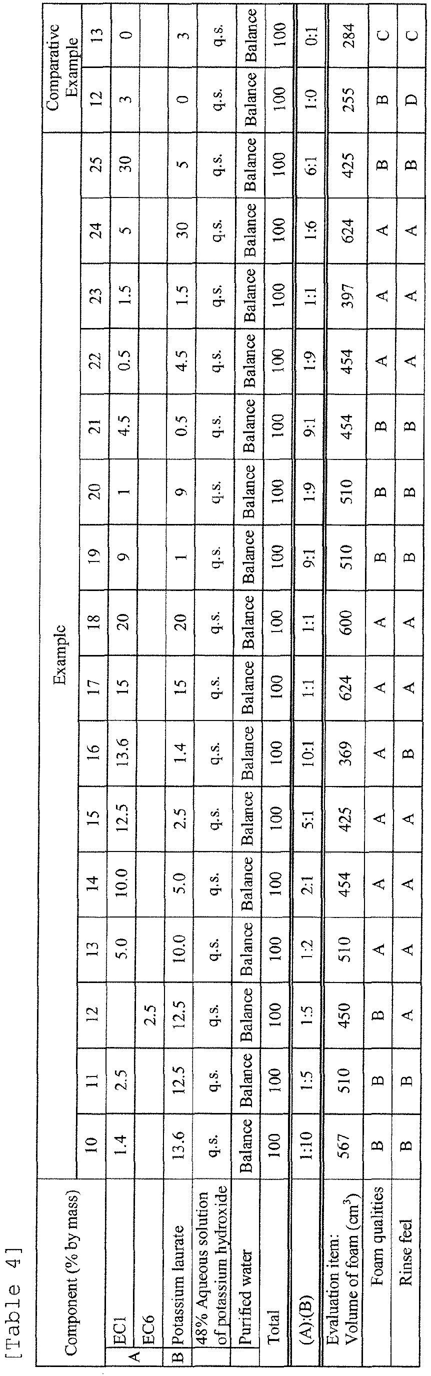

**[Table 5]**

| Component (% by mass) | | Example | | | | | | | | | |
|---|---|---|---|---|---|---|---|---|---|---|---|
| | | 26 | 27 | 28 | 29 | 30 | 31 | 32 | 33 | 34 | 35 |
| A | EC1 | 5.0 | 5.0 | 5.0 | 5.0 | 2.5 | 5.0 | 0.5 | 0.25 | 7.5 | 7.5 |
| B | Potassium laurate | 5.0 | 5.0 | 5.0 | 5.0 | 5.0 | 5.0 | 0.5 | 0.25 | 5.0 | 5.0 |
| c | Lauric acid amidopropyl betaine *5 | 5.0 | | | | | | | | | |
| | Lauryl hydroxy sulfobetaine *6 | | 5.0 | | | | | 1.0 | 0.5 | 15.0 | 20.0 |
| | 2-Alkyl-N-carboxymethyl-N-hydroxyethyl imidazolium betaine *7 | | | 5.0 | | | | | | | |
| | Lauryldimethylamine oxide *8 | | | | 2.5 | | | | | | |
| | Lauryl dimethylaminoacetic acid betaine *9 | | | | | 5.0 | | | | | |
| | 48% Aqueous solution of potassium hydroxide | q.s. | q.s. | q.s. | q.s. | q.s. | q.s. | q.s. | q.s. | q.s. | q.s. |
| | Purified water | Balance | Balance | Balance | Balance | Balance | Balance | Balance | Balance | Balance | Balance |
| | Total | 100 | 100 | 100 | 100 | 100 | 100 | 100 | 100 | 100 | 100 |
| | (A):(C) | 1:1 | 1:1 | 1:1 | 2:1 | 2:1 | - | 1:2 | 2:1 | 1:2 | 1:2 |
| | Evaluation item: Volume of foam (cm³) | 560 | 560 | 550 | 480 | 450 | 400 | 369 | 369 | 600 | 567 |
| | Foam qualities | A | A | A | A | A | A | A | B | A | A |
| | Rinse feel | A | A | A | A | A | A | A | A | B | B |

| | | | | | | | | | | | |
|---|---|---|---|---|---|---|---|---|---|---|---|
| *5: AMPHITOL 20AB (manufactured by Kao Corporation) *6: AMPHITOL 20HD (manufactured by Kao Corporation) *7: AMPHITOL 20YB (manufactured by Kao Corporation) *8: AMPHITOL 20N (manufactured by Kao Corporation) *9: AMPHITOL 20BS (manufactured by Kao Corporation) | | | | | | | | | | | |

**[Table 6]**

| Component (% by mass) | | Example | | | |
|---|---|---|---|---|---|
| | | 36 | 37 | 38 | 39 |
| A | EC1 | 7.5 | 7.5 | 7.5 | 7.5 |
| B | Potassium laurate | 7.5 | 7.5 | 7.5 | 7.5 |
| | 48% Aqueous solution of sodium hydroxide | q.s. | q.s. | q.s. | q.s. |
| | L-Arginine | | 0.5 | | |
| | Triethanolamine | | | 0.5 | |
| | Aminomethyl propanol | | | | 0.5 |
| | Purified water | Balance | Balance | Balance | Balance |
| | Total | 100 | 100 | 100 | 100 |
| | Evaluation item: Volume of foam (cm³) | 397 | 255 | 300 | 255 |
| | Foam qualities | A | A | A | A |
| | Rinse feel | A | B | B | B |

### Test Example 1 (Friction behavior during rinsing)

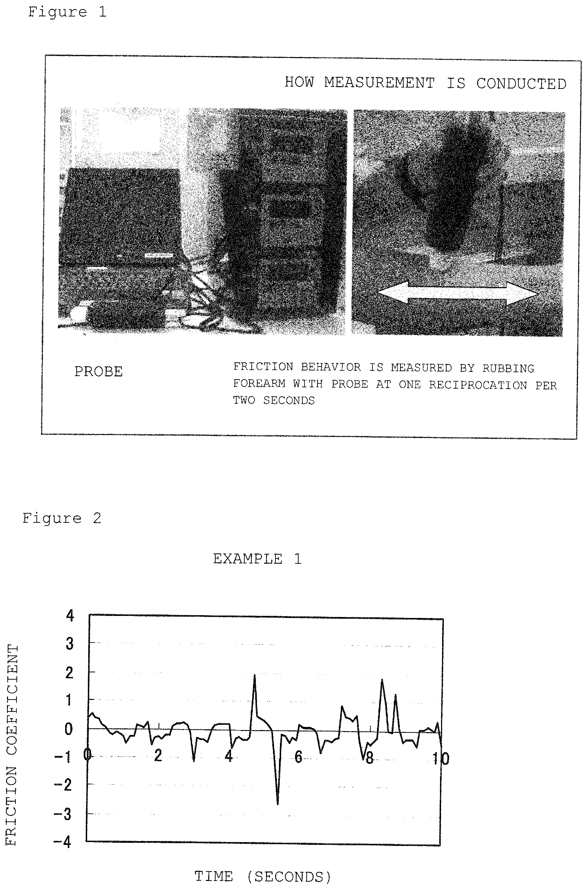

Each cleansing composition (1 g) was placed on the forearm wetted with water. Washing was performed by rubbing the forearm back and forth 10 times with the palm of the other hand, followed by rinsing with tap water (approximately 30°C). At this time, friction behavior of the wet forearm was measured by a portable tactile meter (manufactured by Shinto Scientific Co., Ltd.) as follows (see Figure 1).
(1) Hold a probe perpendicularly on the forearm, with the inside of the arm facing up;
(2) Slide the probe from the elbow side to the wrist of the forearm, and turn it back before the wrist. At this time, adjust the speed of the probe so that it takes approximately two seconds for the probe to travel from the elbow to the wrist, which is approximately 20 cm long. Further, adjust the pressing pressure to approximately 100 N.
(3) (2) was repeated for 10 seconds, and from the friction force thus obtained, friction coefficient was calculated and graphed.

The results of Example 1 and Comparative Examples 2, 8, and 6 are shown in Figures 2 to 5.

The aforementioned Figures will be more specifically explained using schematic diagrams.

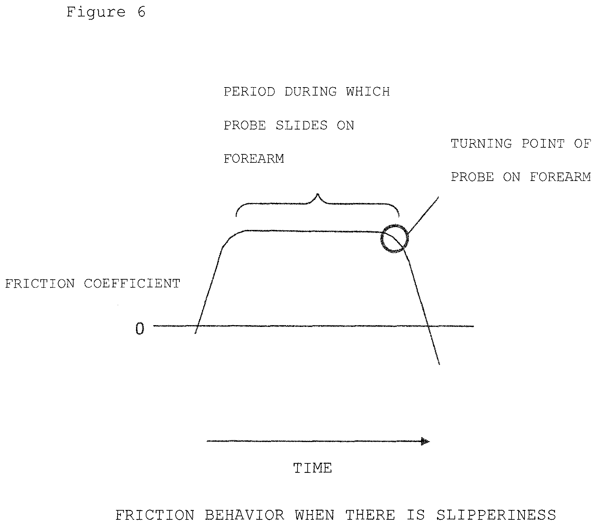

A schematic diagram of friction behavior when there is slipperiness during rinsing is shown in Figure 6. In this case, no clear static friction is observed as the probe starts to slide, and moreover, while the probe is slid, friction coefficient is nearly constant. When friction behavior close to the above is observed, one's hands do not feel stopped throughout the rinsing procedure (rinsing with water while rubbing the skin with hands). As a result, one cannot feel that cleansing compositions are rinsed off, being left with the impression that the cleansing compositions are still left on the skin.

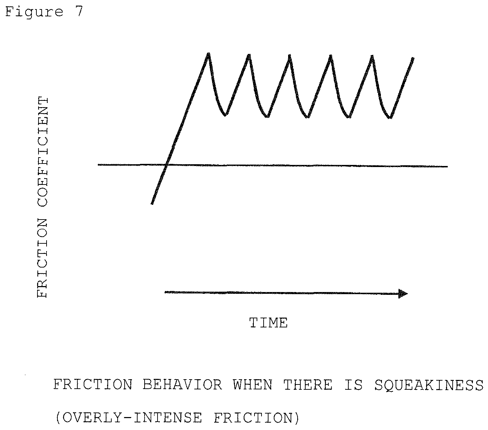

Next, a schematic diagram of friction behavior when there is squeakiness (overly-intense friction) during rinsing is shown in Figure 7. In this case, the following cycle is repeated: hands get caught even while sliding → slide → get caught (a stick-slip-like phenomenon). When the above cycle-like behavior is observed, hands get caught during rinsing, causing consumers to think of a negative impact on the skin, i.e., a feeling that oil on the skin has been completely removed or something is still left on the skin.

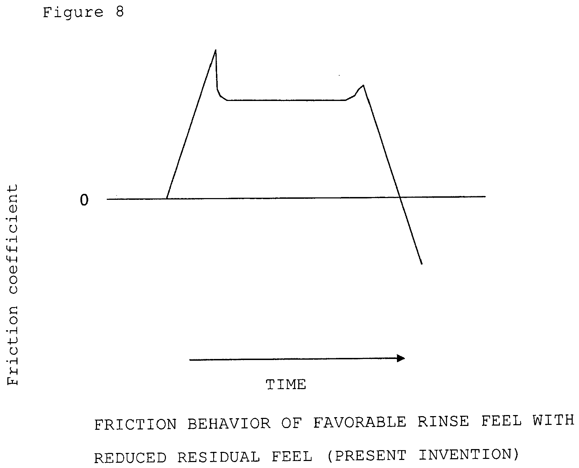

Next, a schematic diagram of friction behavior when favorable rinse feel is obtained with reduced residual feel during rinsing is shown in Figure 8. In this case, clear static friction is observed as the probe starts to slide, and moreover, while the probe is slid, friction coefficient is nearly constant. When behavior close to the above is observed, one's hands feel stopped during rinsing but do not get caught while sliding. As a result, consumers feel that cleansers are completely washed off, and moreover, favorable rinse feel with reduced residual feel is obtained without causing consumers to think of a negative impact on the skin, i.e., a feeling that oil on the skin has been completely removed or something is still left on the skin.

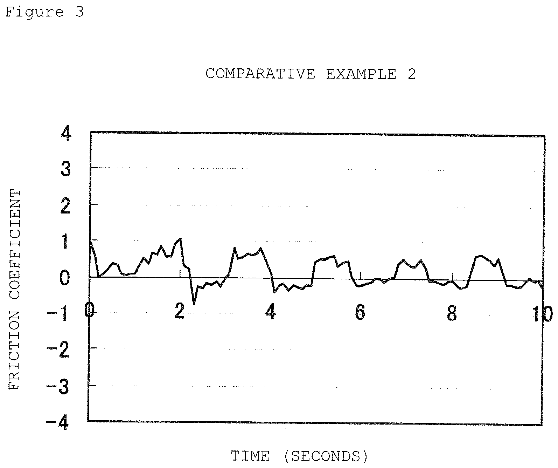

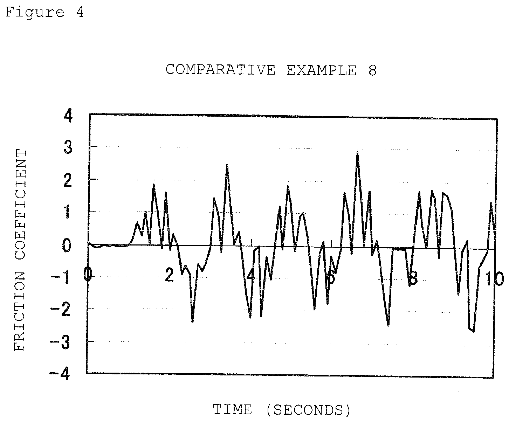

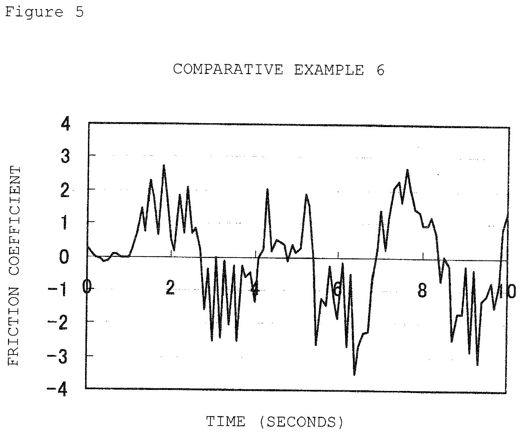

The above results are compared to the aforementioned Figures 2 to 5. Figure 2 shows friction behavior of Example 1 during rinsing. In addition to clear static friction, friction coefficient is nearly constant while the probe is slid, which indicates that friction behavior is close to the schematic diagram of Figure 8. Moreover, also according to the organoleptic evaluation conducted by expert panelists, Example 1 was given an evaluation of "A; While hands move smoothly when they are sliding on the skin, they feel themselves getting caught when they make a turn in a reciprocatory rubbing movement, and non-slippery, excellent rinse feel is obtained without residual feel." Meanwhile, Figure 3 shows friction behavior of Comparative Example 2 during rinsing. The results are close to Figure 6 with no clear static friction observed. Further, also according to the organoleptic evaluation conducted by expert panelists, Comparative Example 2 was given an evaluation of "D; Rinsing was difficult with slipperiness and residual feel." Furthermore, Figures 4 and 5 show friction behavior of Comparative Examples 8 and 6 during rinsing, respectively. Both of them show stick-slip-like friction behavior, and these results are close to Figure 7. Further, also according to the organoleptic evaluation conducted by expert panelists, Comparative Examples 8 and 6 were given an evaluation of "C; Poor rinse feel with an intense sense of friction and residual feel."

The above results confirmed that the present invention obtained a characteristic rinse feel (rinse feel with reduced residual feel).

### Test Example 2 (Friction behavior during rinsing)

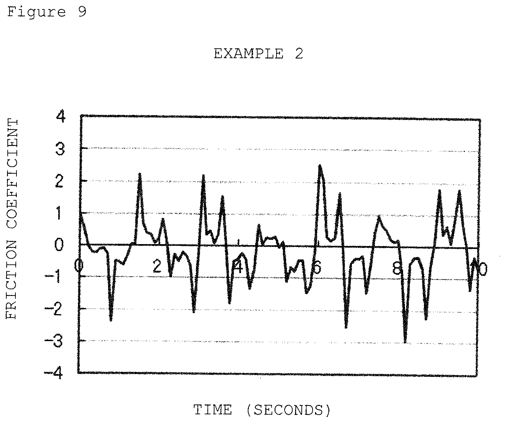

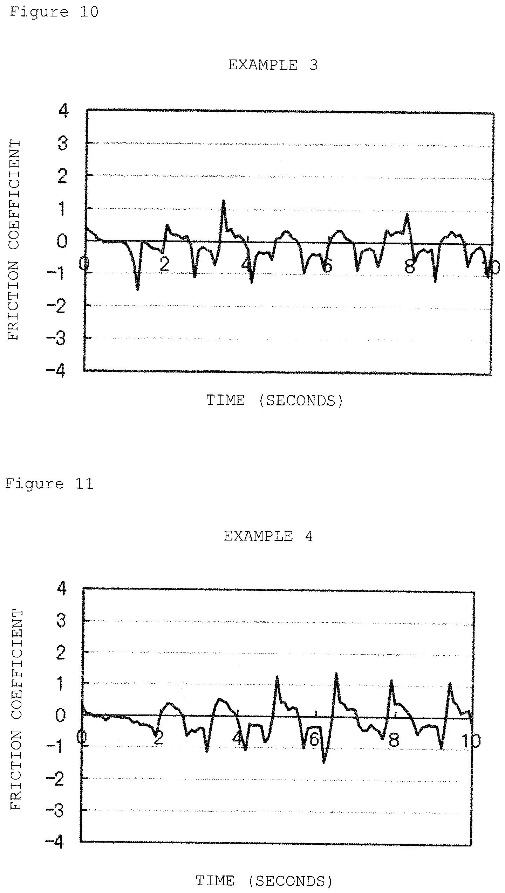

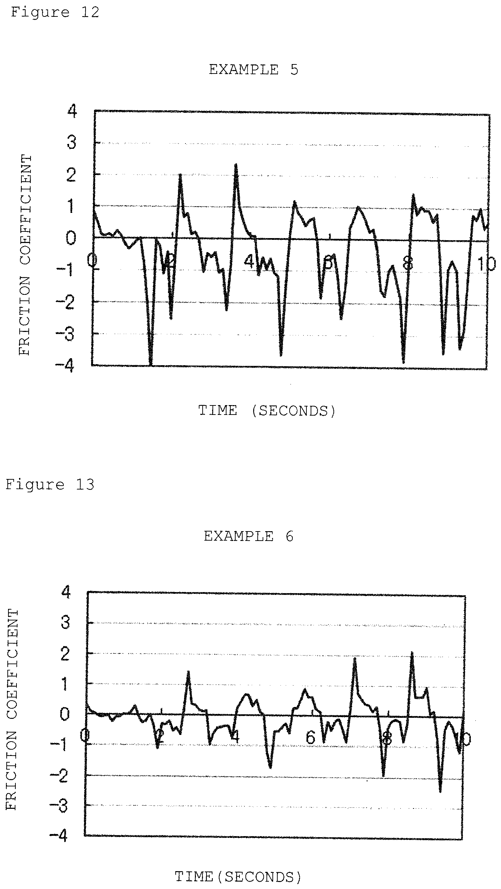

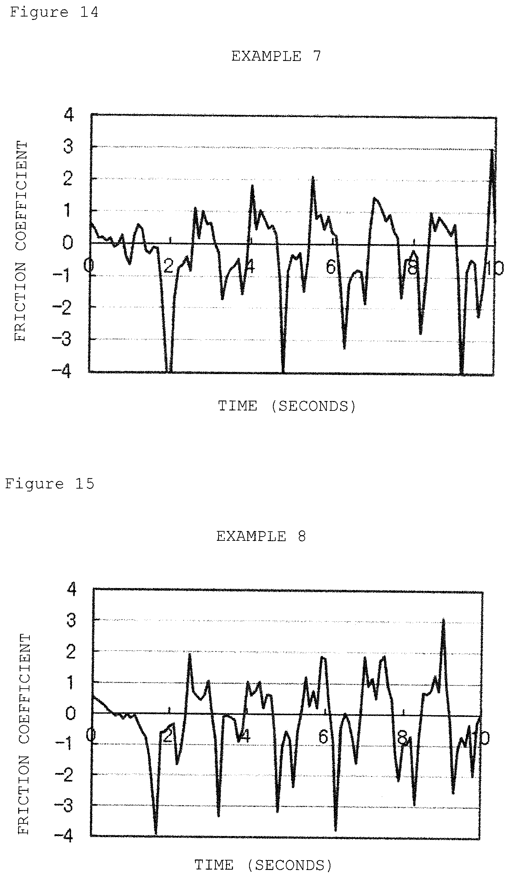

In a similar manner to Test Example 1, the cleansing compositions of Examples 2 to 8 were measured for friction behavior during rinsing. The results are shown in Figures 9 to 15.

From the results of Figures 9 to 15, it was confirmed that, similarly to Example 1, a characteristic rinse feel (rinse feel with reduced residual feel) was also obtainable with the cleansing compositions of Examples 2 to 8.

## Claims

1. A skin cleansing composition, comprising the following components (A) and (B):
(A) from 0.1 to 30% by mass of an alkyl ether carboxylic acid or a salt thereof represented by the formula (1):
R¹-O-(CH₂CH₂O)ₙ-CH₂-COOM (1)
wherein, R¹ represents an alkyl group having 8 to 18 carbon atoms, n represents a number of from 0 to 20, and M represents a hydrogen atom, alkali metal, alkaline earth metal, ammonium, or organic ammonium, wherein R¹ contains two or more alkyl groups, and the content of a component having an alkyl chain length which is contained in the highest content is 55% by mass or more and less than 97% by mass,
wherein, R¹ has an average carbon number of from 10.8 to 12.8 and an average value of n is from 2.5 to 3.4,
and wherein, the alkyl ether carboxylic acid or the salt thereof contains a component in which n = 0 in an amount of from 9.6 to 27% by mass, and a component in which n = 1 and a component in which n = 2 in a total amount of 27% by mass to 36.5% by mass, and
(B) from 0.1 to 30% by mass of a potassium salt or an alkanolamine salt of a fatty acid represented by the formula (2) :
R²-COOY (2)
wherein, R² represents an alkyl group or an alkenyl group having 9 to 22 carbon atoms, and Y represents potassium or alkanolamine.

2. The skin cleansing composition according to claim 1, wherein the content of the component (A) is from 0.5 to 20% by mass.

3. The skin cleansing composition according to claim 1 or 2, wherein the content of the component (A) is from 1 to 15% by mass.

4. The skin cleansing composition according to any one of claims 1 to 3, wherein a mass ratio of the component (A) to the component (B) is (A) : (B) = 1 : 10 to 10 : 1.

5. The skin cleansing composition according to any one of claims 1 to 4, wherein a mass ratio of the component (A) to the component (B) is (A) : (B) = 1 : 5 to 5 : 1.

6. The skin cleansing composition according to any one of claims 1 to 5, wherein a mass ratio of the component (A) to the component (B) is (A) : (B) = 1 : 2 to 2 : 1.

7. The skin cleansing composition according to any one of claims 1 to 6, further comprising (C) an amphoteric surfactant.

8. The skin cleansing composition according to claim 7, wherein a mass ratio of the component (A) to the component (C) is (A) : (C) = 2 : 1 to 1 : 2.

9. A skin cleansing method, comprising applying the skin cleansing composition according to any one of claims 1 to 8 to a skin area, washing, and then rinsing.

10. Use of the composition according to any one of claims 1 to 8 as a skin cleanser.

## Patentansprüche

1. Hautreinigungszusammensetzung, enthaltend die folgenden Komponenten (A) und (B):
(A) von 0,1 bis 30 Masse-% einer Alkylethercarbonsäure oder eines Salzes davon mit der Formel (1):
R¹-O-(CH₂CH₂O)ₙ-CH₂-COOM (1)
worin R¹ eine Alkylgruppe mit 8 bis 18 Kohlenstoffatomen ist, n eine Zahl von 0 bis 20 ist und M ein Wasserstoffatom, Alkalimetall, Erdalkalimetall, Ammonium oder organisches Ammonium ist, worin R¹ zwei oder mehrere Alkylgruppen enthält, und der Gehalt einer Komponente mit einer Alkylkettenlänge, die in dem höchsten Gehalt enthalten ist, 55 Masse-% oder mehr und weniger als 97 Masse-% ist,
worin R¹ eine durchschnittliche Kohlenstoffzahl von 10,8 bis 12,8 hat und ein durchschnittlicher Wert von n von 2,5 bis 3,4 ist,
und worin die Alkylethercarbonsäure oder das Salz davon eine Komponente, worin n = 0, in einer Menge von 9,6 bis 27 Masse-% und eine Komponente, worin n = 1, und eine Komponente, worin n = 2, in einer Gesamtmenge von 27 bis 36,5 Masse-% enthält, und
(B) von 0,1 bis 30 Masse-% eines Kaliumsalzes oder eines Alkanolaminsalzes einer Fettsäure, dargestellt durch die Formel (2):
R²-COOY (2)
worin R² eine Alkylgruppe oder Alkenylgruppe mit 9 bis 22 Kohlenstoffatomen ist und Y Kalium oder Alkanolamin ist.

2. Hautreinigungszusammensetzung gemäß Anspruch 1, worin der Gehalt der Komponente (A) von 0,5 bis 20 Masse-% ist.

3. Hautreinigungszusammensetzung gemäß Anspruch 1 oder 2, worin der Gehalt der Komponente (A) von 1 bis 15 Masse-% ist.

4. Hautreinigungszusammensetzung gemäß einem der Ansprüche 1 bis 3, worin ein Massenverhältnis der Komponente (A) zu der Komponente (B), (A) : (B) = 1 : 10 bis 10 : 1 ist.

5. Hautreinigungszusammensetzung gemäß einem der Ansprüche 1 bis 4, worin ein Massenverhältnis der Komponente (A) zu der Komponente (B), (A) : (B) = 1 : 5 bis 5 : 1 ist.

6. Hautreinigungszusammensetzung gemäß einem der Ansprüche 1 bis 5, worin ein Massenverhältnis der Komponente (A) zu der Komponente (B), (A) : (B) = 1 : 2 bis 2 : 1 ist.

7. Hautreinigungszusammensetzung gemäß einem der Ansprüche 1 bis 6, weiterhin enthaltend (C) ein amphoteres Tensid.

8. Hautreinigungszusammensetzung gemäß Anspruch 7, worin ein Massenverhältnis der Komponente (A) zu der Komponente (C), (A) : (C) = 2 : 1 bis 1 : 2 ist.

9. Hautreinigungsverfahren, enthaltend das Auftragen der Hautreinigungszusammensetzung gemäß einem der Ansprüche 1 bis 8 auf eine Hautfläche, Waschen und anschließendes Spülen.

10. Verwendung der Zusammensetzung gemäß einem der Ansprüche 1 bis 8 als Hautreiniger.

## Revendications

1. Composition nettoyante pour la peau, comprenant les composants suivants (A) et (B) :
(A) de 0,1 à 30 % en masse d'un acide carboxylique d'éther alkylique ou un sel de celui-ci représenté par la formule (1) :
R¹-O-(CH₂CH₂O)ₙ-CH₂-COOM (1)
dans laquelle, R¹ représente un groupe alkyle ayant de 8 à 18 atomes de carbone, n représente un nombre allant de 0 à 20, et M représente un atome d'hydrogène, un métal alcalin, un métal alcalino-terreux, de l'ammonium, ou de l'ammonium organique, dans laquelle R¹ contient deux ou plus de deux groupes alkyle, et la teneur d'un composant ayant une longueur de chaîne alkyle qui figure dans la teneur la plus élevée est de 55 % en masse ou plus et inférieure à 97 % en masse,
dans laquelle, R¹ a un nombre d'atomes de carbone moyen allant de 10,8 à 12,8 et une valeur moyenne de n est de 2,5 à 3,4,
et dans laquelle, l'acide carboxylique d'éther alkylique ou le sel de celui-ci contient un composant dans lequel n = 0 en une quantité allant de 9,6 à 27 % en masse, et un composant dans lequel n = 1 et un composant dans lequel n = 2 en une quantité totale de 27 % en masse à 36,5 % en masse, et
(B) de 0,1 à 30 % en masse d'un sel de potassium ou d'un sel d'alcanolamine d'un acide gras représenté par la formule (2) :
R²-COOY (2)
dans laquelle, R² représente un groupe alkyle ou un groupe alcényle ayant de 9 à 22 atomes de carbone, et Y représente le potassium ou l'alcanolamine.

2. Composition nettoyante pour la peau selon la revendication 1, dans laquelle la teneur du composant (A) est de 0,5 à 20 % en masse.

3. Composition nettoyante pour la peau selon la revendication 1 ou 2, dans laquelle la teneur du composant (A) est de 1 à 15 % en masse.

4. Composition nettoyante pour la peau selon l'une quelconque des revendications 1 à 3, dans laquelle un rapport massique entre le composant (A) et le composant (B) est (A) : (B) = 1 : 10 à 10 : 1.

5. Composition nettoyante pour la peau selon l'une quelconque des revendications 1 à 4, dans laquelle un rapport massique entre le composant (A) et le composant (B) est (A) : (B) = 1 : 5 à 5 : 1.

6. Composition nettoyante pour la peau selon l'une quelconque des revendications 1 à 5, dans laquelle un rapport massique entre le composant (A) et le composant (B) est (A) : (B) = 1 : 2 à 2 : 1.

7. Composition nettoyante pour la peau selon l'une quelconque des revendications 1 à 6, comprenant en outre (C) un tensioactif amphotère.

8. Composition nettoyante pour la peau selon la revendication 7, dans laquelle un rapport massique entre le composant (A) et le composant (C) est (A) : (C) = 2 : 1 à 1 : 2.

9. Procédé de nettoyage pour la peau, comprenant l'application de la composition nettoyante pour la peau selon l'une quelconque des revendications 1 à 8 sur une zone de peau, le lavage, puis le rinçage.

10. Utilisation de la composition selon l'une quelconque des revendications 1 à 8 en tant que nettoyant pour la peau.
